# FASCICULE DE BREVET EUROPEEN

(11) **EP 2 513 289 B1**
(45) Date de publication et mention de la délivrance du brevet: **07.12.2016**
(21) Numéro de dépôt: 10809060.6
(22) Date de dépôt: 10.12.2010
(51) Int. Cl.: B65D 81/26

(54) **CONDITIONNEMENT DE PRODUITS SOLIDES DE LEVURE SANS CARTON**
KARTONFREIE VERPACKUNG VON FESTEN HEFEPRODUKTEN
CARDBOARD-BOX-FREE PACKAGING OF SOLID YEAST PRODUCTS

(30) Priorité: 14.12.2009 FR 0958921
(43) Date de publication de la demande: 24.10.2012
(73) Titulaire: Lesaffre et Compagnie, 75001 Paris (FR)
(72) Inventeur: CHASSARD, Jean-Pierre, F-67113 Blaesheim (FR); LACROIX, Stéphane, F-85440 Talmont Saint Hilaire (FR); STADLER, Bernard, F-67000 Strasbourg (FR); SCHNEIDER, Sophie, F-67114 Eschau (FR)
(74) Mandataire: Cabinet Plasseraud
(86) Numéro de dépôt international: PCT/IB2010/055727
(87) Numéro de publication internationale: WO 2011/073869

(56) Documents cités:
- GB-A- 1 233 902
- US-A- 2 430 459
- US-A- 2 441 477
- US-A- 3 348 673
- US-A- 3 945 493
- US-A- 5 116 191

## Description

### DOMAINE DE L'INVENTION

La présente invention concerne un conditionnement de produits solides de levure. Elle concerne également un procédé de conditionnement de produits solides de levure. Elle concerne également un procédé de conservation et d'utilisation de produits solides de levure.

### ARRIERE-PLAN TECHNIQUE

Les produits de levure, et notamment ceux disponibles sous une forme solide (en particulier la levure pressée), sont particulièrement sensibles à leurs conditions de conservation, notamment à la température, et sont particulièrement exposés aux contaminations. Ce sont ainsi des produits difficiles à conditionner, qui requièrent des conditions de conservation permettant le maintien à la fois de leur qualité microbiologique et de leurs performances (notamment en terme de pouvoir ferment) et ce, afin de garantir la qualité organoleptique des produits de panification préparés à partir de ceux-ci.

En effet, la conservation de produits solides de levure dans des conditions inappropriées ou une variation des conditions de conservation peuvent avoir des conséquences au niveau du métabolisme des levures. Ainsi, l'augmentation du métabolisme fermentaire et / ou respiratoire provoque une production importante de gaz et un échauffement spontané des produits. Il peut en résulter une dégradation de la qualité microbiologique et des performances (notamment en terme de pouvoir ferment) des produits de levure, et donc une dégradation des qualités organoleptiques des produits de panification obtenus à partir de ceux-ci.

En particulier, une augmentation de la température environnante peut entraîner une augmentation du métabolisme des levures, qui a lui-même pour conséquence un dégagement de chaleur supplémentaire. Ainsi une « réaction en chaîne » indésirable est susceptible de se produire ; il convient donc de prévoir des conditionnements de produits solides de levure permettant un refroidissement efficace.

Par exemple, on connaît des produits solides de levure, également appelés « pains », enveloppés individuellement dans du papier ou nus, qui sont regroupés et enveloppés pour former ce que l'on appelle des cartouches, protégées sous cellophane. Typiquement, les cartouches présentent une masse d'approximativement 2,5 kg. Typiquement, les cartouches sont ensuite regroupées par quatre dans des cartons ayant une masse d'approximativement 10 kg chacun. Les cartons sont enfin palettisés en couches croisées formant ainsi un conditionnement.

Etant donné que le carton est un bon isolant thermique, le mode de conditionnement des produits solides de levure actuellement utilisé présente l'inconvénient de ne pas permettre un refroidissement totalement satisfaisant des produits solides de levures.

Afin d'atténuer cet inconvénient, on prévoit généralement des rabats courts sur le dessus des cartons afin de créer une ouverture facilitant le contact avec l'air extérieur; et on se dispense généralement de tout emballage global des cartons sur la palette. Cependant, cela occasionne des risques de contamination des produits. En outre, le niveau de refroidissement des produits solides de levure n'est pas non plus totalement satisfaisant.

Il existe donc un réel besoin de disposer d'un conditionnement apte à la conservation et au maintien des qualités des produits solides de la levure, permettant notamment un refroidissement satisfaisant des produits solides de levure tout en réduisant les risques de contamination de ceux-ci.

US 2 441 477 décrit un emballage multicouches thermo soudé et imperméable à l'eau et à la vapeur d'eau et US 3 945 493 décrit un ensemble d'emballages sur un support et recouvert d'une housse externe.

### RESUME DE L'INVENTION

L'invention concerne en premier lieu un conditionnement de produits solides de levures pressée selon la revendication 1.

Selon un mode de réalisation, la totalité des emballages présents entre les produits solides de levure et la housse externe ont :
- une masse surfacique inférieure ou égale à 100 g/m², et de manière plus particulièrement préférée inférieure ou égale à 80 g/m² ; et / ou
- une épaisseur inférieure ou égale à 150 µm, de préférence inférieure ou égale à 120 µm, de manière plus particulièrement préférée inférieure ou égale à 90 µm.

Selon un mode de réalisation, la housse externe est constituée d'un matériau plastique étirable, de préférence du polyéthylène mono-étiré ou bi-étiré.

Selon un mode de réalisation, les produits solides de levure sont regroupés en cartouches, chaque cartouche étant contenue dans au moins un emballage, et les cartouches sont de préférence regroupées en lots, les cartouches de chaque lot étant de préférence attachées entre elles, dé préférence au moyen d'une ou plusieurs bandes de liaison.

Selon un mode de réalisation :
- des espaces sont ménagés entre au moins une partie des lots ; ou
- l'ensemble des lots sont disposés sans espace entre eux.

Selon un mode de réalisation, les lots sont disposés sur l'élément de support en plusieurs couches superposées.

Selon un mode de réalisation, le conditionnement comprend cinq couches et :
- chaque couche comprend dix lots ;
- chaque lot comprend six cartouches, de préférence réparties en trois rangées de deux cartouches, les cartouches de chaque lot étant de préférence attachées par deux bandes de liaison sensiblement parallèles et deux autres bandes de liaison sensiblement parallèles qui croisent les précédentes ; et
- chaque cartouche comprend cinq produits solides de levure.

Selon un mode de réalisation, les produits solides de levure sont de la levure pressée, de préférence comprenant de 27 à 34% de matière sèche.

L'invention a également pour objet un procédé de conditionnement de produits solides de levure selon la revendication 9.

Selon un mode de réalisation, la totalité des emballages présents entre les produits solides de levure et la housse externe ont :
- une masse surfacique inférieure ou égale à 100 g/m², et de manière plus particulièrement préférée inférieure ou égale à 80 g/m² ; et / ou
- une épaisseur inférieure où égale à 150 µm, de préférence inférieure ou égale à 120 µm, de manière plus particulièrement préférée inférieure ou égale à 90 µm.

Selon un mode de réalisation :
- les emballages sont constitués de matériaux choisis parmi cellophane, le papier, le film plastique alimentaire, le textile, et de préférence parmi le cellophane et le papier ; et / ou
- la housse externe est constituée d'un matériau plastique étirable, de préférence du polyéthylène mono-étiré ou bi-étiré.

Selon un mode de réalisation, le procédé comprend, avant, l'étape de pose sur l'élément de support des produits solides de levure contenus dans les emballages :
- une étape de regroupement des produits solides de levure en cartouches, chaque cartouche étant contenue dans au moins un emballage ; et
- une étape de regroupement des cartouches en lots, les cartouches de chaque lot étant de préférence attachées entre elles, de préférence au moyen d'une ou plusieurs bandes de liaison ;
et, de préférence, l'étape de pose sur l'élément de support des produits solides de levure contenus dans les emballages comprend la pose des lots sur l'élément de support en couches superposées.

Selon un mode de réalisation, lors de la pose sur l'élément de support des produits solides de levure contenus dans les emballages :
- des espaces sont ménagés entre les lots ; ou
- l'ensemble des lots sont disposés sans espace entre eux.

Selon un mode de réalisation, les produits solides de levure sont de la levure pressée, de préférence comprenant de 27 à 34% de matière sèche.

L'invention a également pour objet un procédé de conservation et d'utilisation de produits solides de levure comprenant les étapes suivantes :
- conditionnement de produits solides de levure dans un conditionnement tel que décrit ci-dessus, de préférence selon un procédé de conditionnement décrit ci-dessus ;
- conservation dudit conditionnement de produits solides de levure jusqu'à son utilisation à une température extérieure inférieure ou égale à 15°C, préférentiellement inférieure ou égale à 10°C, et de manière plus particulièrement préférée inférieure ou égale à 5°C ;
- utilisation d'au moins un des produits solides de levure.

Selon un mode de réalisation, les produits solides de levure sont transportés au cours de l'étape de conservation ; et l'étape de conservation présente de préférence une durée comprise entre 1 jour et 7 semaines, de préférence entre 1 semaine et 7 semaines.

Selon un mode de réalisation, la température de chaque produit solide de levure reste inférieure ou égale à 15°C, de préférence inférieure ou égale à 12°C, et de manière plus particulièrement préférée inférieure ou égale à 10°C lors de l'étape de conservation.

Selon un mode de réalisation, au moins un produit solide de levure est utilisé pour la fabrication d'un produit de panification.

La présente invention permet de surmonter les inconvénients dé l'état de la technique. Elle fournit plus particulièrement un conditionnement de produits solides de levure qui permet le refroidissement des produits solides de levure pendant leur conservation, en même temps qu'il réduit les risques de contamination de ceux-ci. Le maintien de la qualité microbiologique et des performances de ces produits (notamment pouvoir ferment) est ainsi obtenu, de même que le maintien des qualités organoleptiques des produits de panification fabriqués à partir de ceux-ci.

Cela est accompli grâce à un conditionnement de produits solides de levure sans carton, c'est-à-dire utilisant uniquement des emballages relativement fins, et dans lequel l'ensemble des produits de solides de levure emballés sont maintenus sur un élément de support à l'aide d'une housse externe.

Ainsi, le conditionnement selon l'invention offre une isolation thermique inférieure à l'état de la technique, vis-à-vis des produits solides de levure. Il permet donc un meilleur refroidissement des produits solides de levure tout en évitant les problèmes de contamination.

Selon certains modes de réalisation particuliers, l'invention présente également une ou de préférence plusieurs des caractéristiques avantageuses énumérées ci-dessous.
- Dans les conditionnements de l'état de la technique, il est indispensable de prévoir des espaces entre les cartons pour former des couloirs de refroidissement assurant un échange de chaleur entre les produits solides de levure contenus dans les cartons et l'air passant dans les couloirs. L'invention permet de se dispenser si on le souhaite des couloirs de refroidissement, ce qui permet d'augmenter la stabilité du conditionnement, en particulier lors du transport.
- Dans les conditionnements de l'état de la technique, il est indispensable de prévoir un collage des lots de produits entre eux et sur la palette (avec de la colle de palettisation), afin de maintenir l'ensemble des lots en place sur la palette. Or, la présence de colle accroît les risques de contamination des produits. L'intention permet de se dispenser de tout collage des lots de produits entre eux et sur la palette et limite donc les risques de contamination.
- Un conditionnement utilisant du carton nécessite pour le client un déballage et un pliage des cartons. Les cartons doivent ensuite être stockés, acheminés à un point de collecte, compactés et les enlèvements doivent être suivis. Ces opérations génèrent des coûts de main d'oeuvre et de traitement de déchets, fortement réduits par l'utilisation de conditionnements selon l'invention.
- Les enveloppes de conditionnement en matériaux à masse surfacique élevée tels que le carton ne concourent pas à la réduction des emballages conformément à la législation en vigueur. Le conditionnement de l'invention permet donc une meilleure protection de l'environnement que les conditionnements de l'état de la technique.
- Dans les unités de production alimentaire, le carton est critiqué comme étant un facteur potentiel de contaminations croisées. Il ne répond bien souvent pas aux normes sanitaires imposées aux utilisateurs des produits solides de levure, notamment les industriels de la boulangerie. Le conditionnement de l'invention garantit, de par l'absence de l'utilisation de carton, une meilleure hygiène.

### BREVE DESCRIPTION DES FIGURES

La **figure 1** représente un schéma d'un exemple de conditionnement selon l'invéntion.
La **figure 2** représente un schéma d'un détail d'un conditionnement selon l'invention.
Les **figures 3-5** représentent des résultats de tests de température opérés sur différents conditionnements lors de leur conservation. En abscisse figure la date et l'heure des mesure et en ordonnée figure le résultat de la mesure de température, en degrés Celsius.

### DESCRIPTION DE MODES DE REALISATION DE L'INVENTION

L'invention est maintenant décrite plus en détail et de façon non limitative dans la description qui suit.

L'invention concerne un conditionnement de produits solides de levure. On entend dans toute la suite par «produit solide de levure » tout produit contenant des levures, qui a tendance à conserver essentiellement sa forme même en dehors de tout récipient. Les produits solides de levure se distinguent ainsi des produits liquides de levure (suspension de levures, crème de levure...) et des produits de levure pulvérulents (levure séchée). De préférence, les produits solides de levure contiennent des levures vivantes.

Les produits solides de levure se présentent généralement sous la forme de blocs compacts. Les produits solides de levure peuvent être des pains de levure pressée. Les produits solides de levure contiennent de préférence de 27 à 34% de matière sèche.

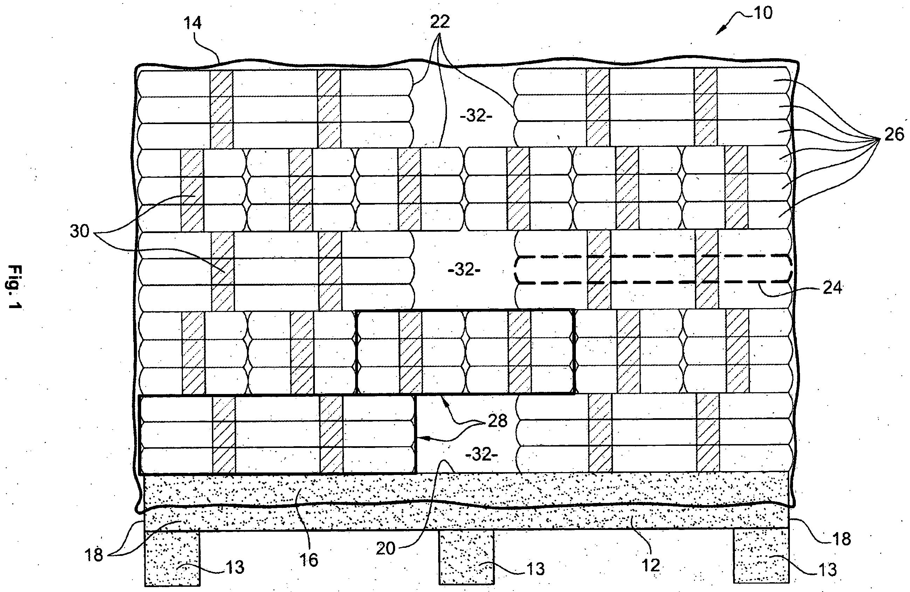

Il est maintenant d'abord fait référence à la **figure 1****,** qui présente un schéma d'un exemple de conditionnement 10 selon l'invention vu de face.

Le conditionnement 10 de produits solides de levure comprend un élément de support 12. L'élément de support 12 permet le support des produits solides de levure. Il peut présenter des pieds d'appui 13. Il comprend en outre en général une plaque de support ou, selon un mode de réalisation préféré, un ensemble de barres ou planches transversales et longitudinales attachées les unes ou autres. Cette deuxième solution assure un passage de l'air par le côté inférieur du conditionnement (entre les barres de l'élément de support 12) et permet donc un meilleur refroidissement des produits solides de levure.

L'élément de support peut être de forme générale parallélépipédique, ce qui permet un rangement optimal du conditionnement lorsqu'il est stocké avec d'autres conditionnements. Il peut s'agir d'une palette de manutention, ce qui permet le transport du conditionnement par un chariot adapté au transport de palettes (chariot transpalette). L'élément de support 12 peut être en bois. Un élément de support en bois présente l'avantage d'un coût de production faible. Le bois peut également permettre une certaine absorption de l'humidité. L'élément de support 12 peut être en matière plastique, ce qui peut être avantageux en cas de condensation importante, puisque le plastique n'est pas affecté par la présence d'eau.

Le conditionnement 10 comprend une housse externe 14 maintenant l'ensemble des produits solides de levure sur l'élément de support 12. Cette housse externe 14 peut être un film plastique appliqué autour de l'ensemble des produits solides de levure de manière à exercer sur ceux-ci une pression les maintenant sur l'élément de support 12, et éventuellement les maintenant serrés entre eux.

L'empilement des produits solides de levure sur l'élément de support 12 définissant une surface supérieure et une surface latérale (cette dernière étant par exemple composée de quatre faces si l'empilement est de forme essentiellement parallélépipédique), la housse externe 14 recouvre l'ensemble de la surface latérale de l'empilement et éventuellement recouvre également tout ou partie de la surface supérieure de celui-ci.

La housse externe 14 peut également être appliquée sur une partie 16 de l'élément de support 12. Si l'élément de support 12 est de forme générale parallélépipédique, l'emballage peut s'appliquer sur une partie 16 supérieure des quatre faces 18 latérales de l'élément de support 12, les produits solides de levure étant supportés par la face supérieure 20 de l'élément de support 12. Alternativement, on peut prévoir des bandes de liaison entourant la housse externe 14 et l'élément de support 12 afin de les attacher ensemble.

La housse externe 14 peut notamment être tout film plastique prévu pour le conditionnement des produits alimentaires. La housse externe 14 peut être en un matériau étirable, de préférence un matériau plastique étirable. Il peut notamment s'agir de polyéthylène et en particulier de polyéthylène mono-étiré ou bi-étiré. La housse externe 14 peut être thermorétractable. La housse externe 14 peut également être tout film utilisé dans les procédés de palettisation connus de l'art antérieur. Selon un mode de réalisation particulier, la housse externe 14 est constituée d'un film plastique relativement perméable aux gaz et en particulier à la vapeur d'eau, afin d'éviter la condensation de l'eau sur la surface de la housse et afin de permettre un meilleur refroidissement des produits.

Le conditionnement 10 comprend un ou plusieurs emballages 22 entre les produits solides de levures et la housse externe 14. Un emballage 22 désigne toute structure qui entoure ou enveloppe un produit de levure ou plusieurs produits de levure (eux-mêmes déjà emballés ou non), c'est-à-dire qui les isole ou les sépare de l'extérieur.

Un exemple 24 d'une surface d'emballage 22 est mis en évidence en traits pointillés gras sur la **figure 1****.** Les produits solides de levure individuels qui sont à l'intérieur des emballages 22 ne sont pas visibles sur la **figure 1****.**

Les emballages peuvent être constitués de tout matériau solide sous forme de feuille ou de film, susceptible de protéger les produits solides de levure de tout contact avec l'environnement. Ce matériau est en partie perméable aux gaz, voire aux liquides pour éviter les problèmes de gonflement (dus à l'émission de gaz) ou de condensation d'eau. De préférence, les emballages 22 sont complètement fermés (et en particulier sont étanches aux micro-organismes). Les emballages 22 sont de préférence souples, mais ils peuvent éventuellement être rigides.

Un emballage 22 peut contenir directement un ou plusieurs produits solides de levure et / ou contenir un ou plusieurs autres emballages 22. Ainsi on peut par exemple grouper plusieurs produits solides de levure entre eux dans au moins un emballage, et réunir plusieurs de ces groupes entre eux dans au moins un autre emballage.

Chaque produit solide de levure est ainsi contenu dans, ou entouré par, au moins un emballage 22, et éventuellement plusieurs.

Ainsi l'élément de support 12 ne supporte pas directement les produits solides de levure mais il supporte les emballages 22 qui les contiennent. De même, la housse externe 14 ne maintient pas directement les produits solides de levure sur l'élément de support 12, mais elle maintient les emballages 22 qui les contiennent.

Tous les emballages 22 entre les produits solides de levure et la housse externe 14 présentent une masse surfacique (ou grammage) inférieure ou égale à 150 g/m². En d'autres termes, chaque emballage 22 situé à l'intérieur de la housse externe 14, et chaque structure assimilable à un emballage 22 (selon les définitions données ci-dessus) présente une masse surfacique inférieure à 150 g/m².

De manière additive, tous les emballages 22 entre les produits solides dé levure et la housse externe 14 peuvent présenter une épaisseur inférieure ou égale à 150 µm. En d'autres termes, chaque emballage 22 situé à l'intérieur de la housse externe 14, et chaque structure assimilable à un emballage 22 (selon les définirions données ci-dessus) présente une épaisseur inférieure ou égale à 150 µm.

Autrement dit, aucun emballage de masse surfacique supérieure à 150 g/m² n'est présent à l'intérieur de la housse externe 14 ; en particulier, aucun emballage en carton n'est présent à l'intérieur de la housse externe 14 (le carton étant défini par une masse surfacique supérieure à 224 g/m²). Ou, en particulier, aucun emballage en carton n'est présent à l'intérieur de la housse externe 14 (le carton étant défini par une épaisseur supérieure à 175 µm).

De préférence, la housse externe 14 présente les mêmes caractéristiques de masse surfacique (ou d'épaisseur) que les emballages 22.

Grâce au faible grammage (ou à la faible épaisseur) des emballages 22, on permet un bon échange thermique entre les produits solides de levure et l'air extérieur, et donc un bon refroidissement des produits solides de levure. Ce meilleur refroidissement est associé à la possibilité d'isoler les produits solides de levure de manière plus complète que dans l'art antérieur (les cartons étant ouverts, dans l'art intérieur). Cela permet de diminuer les risques de contamination des produits.

En outre, les couloirs de refroidissement entre les emballages 22 sont possibles mais ne sont pas indispensables selon l'invention, à la différence de l'état de la technique. L'invention permet plus généralement d'éviter l'ensemble des inconvénients mentionnés ci-dessus liés à la présence de carton.

La housse externe 14 protège avantageusement les produits solides de levure de toute contamination ; l'utilisation de cette housse externe 14 enveloppant tous les produits est rendue possible par l'absence d'emballage de grammage élevé (carton) dans le conditionnement.

De manière générale, plus le grammage des emballages 22 est faible, et / ou plus l'épaisseur des emballages 22 est faible, plus les avantages mentionnés ci-dessus sont accentués. Ainsi, la totalité des emballages 22 entre les produits solides de levure et la housse externe 14 peuvent présenter une masse surfacique inférieure ou égale à 120 g/m², préférentiellement inférieure ou égale à 100 g/m², préférentiellement inférieure ou égale à 80 g/m², préférentiellement inférieure ou égale, à 70 g/m², préférentiellement inférieure ou égale à 60 g/m², préférentiellement inférieure ou égale à 55 g/m², préférentiellement inférieure ou égale à 50 g/m², préférentiellement inférieure ou égale à 45 g/m². De manière additive, la totalité des emballages 22 entre les produits solides de levure et l'emballage 14 peuvent présenter une épaisseur inférieure ou égale à 120 µm, préférentiellement inférieure ou égale à 90 µm, préférentiellement inférieure ou égale à 70 µm, préférentiellement inférieure ou égale à 60 µm, préférentiellement inférieure ou égale à 50 µm, préférentiellement inférieure ou égale à 40 µm, préférentiellement inférieure ou égale à 35 µm.

De manière générale, plus le grammage des emballages 22 est élevé, et / ou plus l'épaisseur des emballages 22 est élevée,-plus leur rigidité est élevée. Ainsi, tous les emballages 22 entre les produits solides de levure et la housse externe 14 peuvent présenter une masse surfacique supérieure ou égale à 5 g/m², préférentiellement supérieure ou égale à 10 g/m², préférentiellement supérieure ou égale à 15 g/m², préférentiellement supérieure ou égale à 20 g/m². Alternativement ou de manière additive, tous les emballages 22 entre les produits solides de levure et la housse externe 14 peuvent présenter une épaisseur supérieure ou égale à 5 micromètres, préférentiellement supérieure ou égale à 10 micromètres, préférentiellement supérieure ou égale à 15 micromètres, préférentiellement supérieure ou égale à 20 micromètres.

Les emballages 22 peuvent être constitués de cellophane et / ou de papier. En effet, ces matériaux présentent une rigidité satisfaisante malgré une faible masse surfacique et une faible épaisseur. Ils sont étanches aux micro-organismes, et semi-perméables aux gaz et liquides. La cellophane peut être transparente, ce qui peut permettre d'inspecter visuellement les produits solides de levure sans les retirer de l'emballage. C'est en outre un matériau avantageux car il n'est pas affecté par l'humidité. Alternativement, les emballages 22 peuvent être en matière plastique alimentaire partiellement perméable ou en textile. Typiquement, les produits solides de levure peuvent être répartis dans des emballages 22 uniques en cellophane, ou dans deux emballages 22 superposés (concentriques), respectivement en cellophane et en papier.

Dans le mode de réalisation de la **figure 1****,** les produits solides de levure sont regroupés en cartouches 26, chaque cartouche 26 étant dans un emballage 22 (ou dans deux emballages 22 superposés). Les cartouches 26 sont regroupées en lots 28 afin d'augmenter la stabilité du conditionnement. Chaque lot 28 peut être contenu dans un emballage, ou bien de préférence, comme cela est représenté sur la **figure 1****,** les lots 28 peuvent être formés par attachement des cartouches 26 au moyen d'une ou plusieurs bandes de liaison 30 : ainsi on évite la présence d'un emballage supplémentaire et on facilite le refroidissement des produits. Dans l'exemple de la **figure 1****,** deux des lots 28 ont été mis en évidence par un trait gras.

Une bande de liaison est un moyen d'attache long et étroit qui peut se refermer de manière à ceinturer plusieurs cartouches 26. Parce qu'elle ne recouvre pas complètement la surface du lot 28, la bande de liaison 30 n'est pas considérée comme un emballage 22. Les bandes de liaison 30 peuvent être par exemple en papier, en matière plastique, en carton ou en textile. Les bandes de liaison 30 sont de préférence constituées d'un matériau résistant à l'humidité. Les bandes de liaison 30 sont suffisamment étroites pour ne pas gêner le refroidissement des produits solides de levure. Attacher les cartouches 26 avec des bandes de liaison 30 permet de mieux transporter les lots 28 de cartouches 26 ainsi constitués, les bandes de liaison 30 formant alors des poignées. Les bandes de liaison 30 peuvent prévoir des moyens assurant leur enlèvement rapide par l'utilisateur qui n'a ainsi pas à utiliser d'outils spécifiques. Les bandes de liaison 30 peuvent être rigides afin d'éviter de former une empreinte sur les produits solides de levures.

Les produits solides de levure peuvent être regroupés en cartouches de manière que les cartouches 26 présentent une forme sensiblement parallélépipédique avec une longueur au moins une fois et demie, préférentiellement au moins deux fois, supérieure à la largeur et/ou à la hauteur. Dans ce cas, les cartouches 26 sont attachées en lots 28 de manière à ce que toutes les longueurs des cartouches 26 d'un lot 28 soient dans la même direction. Cette configuration confère une bonne stabilité à un lot 28.

Les cartouches 26 peuvent comporter une soudure, notamment si les emballages 22 sont en cellophane. Les cartouches peuvent alors être positionnés tête-bêche dans les lots 28 de manière à ce que les soudures de deux cartouches 26 ne soient pas en contact. Cela évite que les frottements entre des emballages 22 adjacents n'ouvrent les soudures lors du transport du conditionnement 10.

Comme cela est le cas dans l'exemple de la **figure 1****,** des espaces 32 peuvent être prévus entre les lots 28. Ces espaces 32 (couloirs de refroidissement) peuvent améliorer le refroidissement des produits solides de levure.

Inversement, les lots 28 peuvent être serrés les uns contre les autres. Cette configuration permet une utilisation optimale de la surface offerte par l'élément de support 12 et permet une plus grande stabilité lors du transport du conditionnement 10.

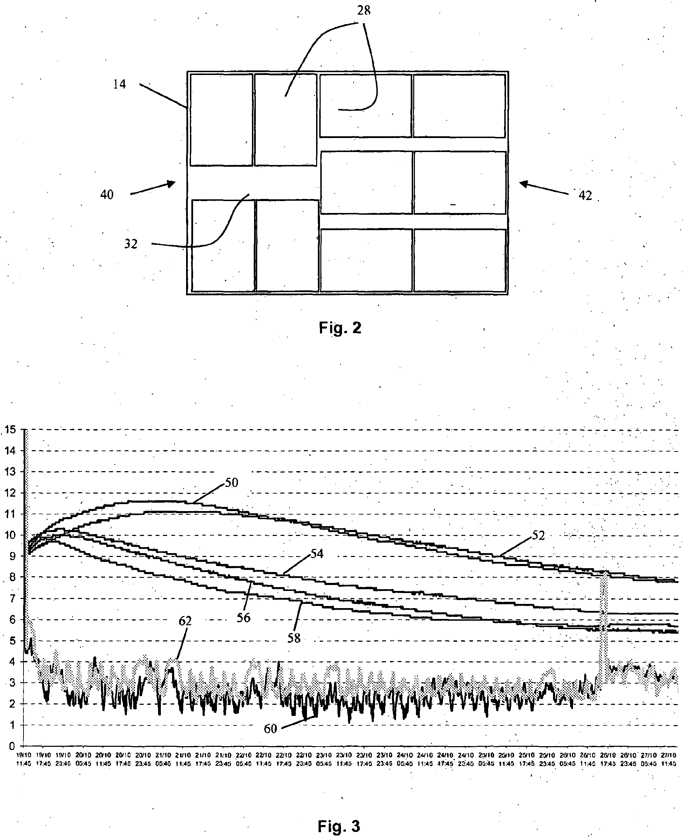

Comme cela est le cas dans l'exemple de la **figure 1****,** les lots 28 peuvent être disposés sur l'élément de support 12 en plusieurs couches. Cela permet une meilleure utilisation de l'espace au dessus de l'élément de support 12. Un exemple de conditionnement avec dix lots 28 par couche est représenté sur la **figure 2****,** qui est un schéma d'une couche vue de dessus.

Dans cet exemple, quatre lots 28 sont disposés longitudinalement dans la direction de la largeur du conditionnement, dans une première partie 40 de la couche. Les six autres lots 28 sont disposés longitudinalement dans la direction de la longueur du conditionnement, dans une deuxième partie 42 de la couche. Un espace 32 est ici prévu dans la première partie 40, entre les lots 28.

Comme on peut l'observer sur la **figure 1****,** les couches du conditionnement peuvent être croisées. En d'autres termes, la première partie 40 d'une couche inférieure est disposée directement en dessous de la deuxième partie 42 de la couche située directement au-dessus à cette couche inférieure et réciproquement. Cela implique une alternance dés configurations de couche dans la direction verticale. Cela assure que tous les lots soient en contact avec un espace 32 de refroidissement (lorsque tels espaces sont présents). Cela assure également une meilleure stabilité du conditionnement.

Les cartouches 26 peuvent par exemple regrouper de 4 à 6 produits solides de levure et peuvent présenter une masse de 2 à 3 kg. Les lots 28 peuvent par exemple présenter une masse de 10 à 20 kg. Le conditionnement 10 peut par exemple présenter une masse totale de 700 à 800 kg. La masse totale peut toutefois dépasser les 800 kg, notamment dans le cas où le conditionnement comprend plus de cinq couches.

Dans l'exemple de la **figure 1****,** le conditionnement 10 (d'environ 750 kg) comprend cinq couches. Chaque cartouche 26 (d'environ 2,5 kg) comprend cinq produits solides de levure (pains de levure d'environ 500 g chacun). Chaque lot 28 (d'environ 15 kg) comprend six cartouches 26, de préférence disposées en trois rangées de deux cartouches. Les cartouches 26 d'un lot 28 sont de préférence attachées par deux bandes de liaison 30 sensiblement parallèles et deux autres bandes de liaison 30 sensiblement parallèles qui croisent les précédentes. Chaque couche comprend dix lots 28. Cette configuration du conditionnement 10 constitue un bon compromis entre maniabilité, utilisation optimale de l'espace et stabilité.

Un élément intercalaire inférieur peut être prévu entre l'élément de support 12 et la couche la plus basse des lots 28. Un élément intercalaire supérieur peut être prévu entre le dessus de la housse externe 14 et la couche la plus haute des lots 28. Ces éléments intercalaires ne sont pas considérés comme des emballages 22 au sens de l'invenfion.

De tels éléments intercalaires peuvent être rigides et permettent de solidifier le conditionnement 10, ce qui est particulièrement utile lors de son transport. De tels éléments intercalaires peuvent également être prévus entre les différentes couches de lots 28, permettant ainsi une meilleure stabilisation du conditionnement 10, notamment lorsque des espaces 32 sont prévus entres les lots 28. Ces différents éléments intercalaires peuvent également être prévus pour absorber l'humidité ou résister à l'humidité. Par exemple, les éléments intercalaires peuvent être en plastique ; de préférence, ils présentent une faible capacité d'isolation thermique pour ne pas compromettre le refroidissement des produits.

Le conditionnement 10 décrit ci-dessus peut être obtenu par un procédé de conditionnement de produits solides de levure comprenant les étapes suivantes :
- la fourniture d'un élément de support 12 ;
- l'emballage de produits solides de levure dans un ou plusieurs emballages 22 ;
- la pose sur l'élément due support 12 des produits solides de levure contenus dans les emballages 22 ; et
- la pose d'une housse externe 14 autour de l'ensemble des produits de solides de levure contenus dans les emballages 22.

Le procédé peut comprendre en outre une étape de regroupement des produits solides de levure en cartouches 26 préalablement à leur emballage dans au moins Un emballage 22.

Le procédé peut comprendre en outre une étape d'attachement des cartouches 26 en lots 28, de préférence au moyen d'une ou plusieurs bandes de liaison 30, préalablement à la pose sur l'élément de support 12 des produits solides de levure.

Lors de la pose sur l'élément de support 12 des produits solides de levure, des espaces 32 peuvent être prévus entre les lots 28. Cela peut être réalisé par une disposition particulière des lots 28 sur l'élément de support 12. Inversement, lors de la pose sur l'élément de support 12 des produits solides de levure; les lots 28 peuvent être serrés les uns contre les autres.

Lors de la pose sur l'élément de support 12 des produits solides de levure, les lots 28 peuvent être disposés sur l'élément de support 12 en plusieurs couches.

Les différentes étapes du conditionnement peuvent être réalisées à l'aide d'outils de palettisation connus de l'art antérieur. Il est également à noter qu'au moins une partie des étapes d'un tel procédé de conditionnement peuvent être réalisées en chambre froide, c'est-à-dire avec une température extérieure inférieure ou égale à 15°C, préférablement inférieure ou égale à 10°C, et notamment inférieure ou égale à 5°C. En particulier, l'étape finale de pose de la housse externe 14 peut être effectuée en chambre froide ou avant l'entrée en chambre froids.

Après l'exécution d'un tel procédé de conditionnement, le conditionnement 10 obtenu peut être conservé jusqu'à son utilisation à une température de conservation inférieure ou égale à 15°C, préférentiellement inférieure ou égale à 10°C, préférentiellement inférieure ou égale à 5°C.

La température de conservation est la température extérieure à laquelle est soumis le conditionnement (température de la chambre froide) pendant au moins 90% du temps, de préférence pendant au moins 95% ou pendant au moins 99% du temps. Il n'est donc pas exclu que le conditionnement soit brièvement soumis à une température supérieure aux valeurs indiquées ci-dessus, par exemple s'il est déplacé d'une chambre froide à une autre.

L'étape de conservation du conditionnement peut comprendre le transport du conditionnement (à la température de conservation indiquée ci-dessus).

La durée de la conservation (jusqu'à l'utilisation) est de préférence une durée comprise entre 1 jour et 7 semaines, plus particulièrement entre 1 semaine et 7 semaines.

L'invention permet avantageusement de conserver chaque produit solide de levure dans le conditionnement à une température (locale ou réelle) inférieure ou égale à 15°C, de préférence inférieure ou égale à 12°C, et de manière plus particulièrement préférée inférieure ou égalé à 10°C pendant toute la durée de la conservation.

Les produits solides de levure peuvent être utilisés pour la réalisation de produits de panification et notamment pour la fabrication de pain, de pâtisseries, de viennoiseries, de pâte à pizza...

### EXEMPLES

Les exemples suivants illustrent l'invention sans la limiter.

### Exemple 1 suivi de la température sur des conditionnements selon l'invention

### 1. Matériel

Dans cet essai, deux conditionnements selon l'invention sont utilisés, et l'évolution de la température locale à l'intérieur des conditionnements a été mesurée lors de la conservation des conditionnements en chambre froide grâce à des sondes de température.

Le premier conditionnement est conforme aux **figures 1** **et** **2****,** avec un élément de support en plastique. Le conditionnement comprend cinq couches de dix lots, les lots comprenant six cartouches chacun (en trois rangées de deux cartouches). Les cartouches d'un lot sont attachées par quatre bandes croisées. Chaque cartouche comprend cinq produits solides de levure, et présente une masse d'approximativement 2,5 kg. Des espaces sont ménagés entre les lots (couloirs de refroidissement).

Le deuxième conditionnement , est identique au premier mais ne présente pas de couloirs de refroidissement. En d'autres termes, les lots sont serrés les uns contre les autres.

Les deux conditionnements sont pourvus d'une housse externe constituée de film plastique recouvrant totalement les lots.

La house externe est en polyéthylène linéaire basse densité présentant les caractéristiques suivantes : épaisseur de 23,3 µm, module élastique de 85,5 N/mm², résistance au déchirement de 3000 mN.

La cellophane utilisée présente un grammage de 33,5 g/m² et une épaisseur de 23,3 µm (il serait également possible de la remplacer par une cellophane de 43 g/m² et 29,9 µm d'épaisseur).

Les deux conditionnements sont respectivement entreposés en chambre froide et la température des conditionnements est suivie pendant huit jours. Dans la suite, les couches des conditionnements sont numérotées dans l'ordre croissant de bas en haut (la première couche étant en contact avec l'élément de support).

### 2. Résultats

Les courbes d'évolution de la température en fonction du temps sont représentées sur la **figure 3****.** Le tableau suivant donne un récapitulatif des endroits où la température à été mesurée :

| Référence sur la figure | Nature de la courbe |
|---|---|
| 50 | Température au coeur du premier conditionnement |
| 52 | Température au coeur du deuxième conditionnement |
| 54 | Température dans la cinquième couche du deuxième conditionnement |
| 56 | Température dans la première couche du deuxième conditionnement |
| 58 | Température dans la première couche du premier conditionnement |
| 60 | Température ambiante de la première chambre |
| 62 | Température ambiante de la deuxième chambre |

On observe que la température locale dans le conditionnement (et donc la température des produits solides de levure) est maintenue de façon satisfaisante à un niveau inférieur à 12°C (et en grande partie inférieur à 10°C) tout au long de la conservation à chaque position dans le conditionnement.

En outre, le refroidissement obtenu ne dépend essentiellement pas de la présence ou non des couloirs de refroidissement.

### Exemple 2 (comparatif) : suivi de la température sur des conditionnements selon l'état de la technique

### 1. Matériel

Des essais ont été réalisés afin de suivre l'évolution de la température locale à l'intérieure de conditionnements utilisant des emballages en carton, et conservés en chambre froide pendant huit jours.

Ces conditionnements sont organisés de manière similaire aux conditionnements de l'exemple 1, à ceci près qu'ils présentent dix couches de cartons, contenant chacun quatre cartouches.

Les cartons utilisés sont des cartons Kaysersberg de composition 140×B140×180, de grammage 501,8 g/m², de résistance à l'éclatement 730 kPa, d'ECT 5,4 kN/m, d'épaisseur 2,8 mm et des cartons Smurfit de composition 200×140×140, de grammage 522 g/m², de résistance à l'éclatement 1515 kPa, d'ECT 4,8 kN/m, d'épaisseur 2,9 mm.

Les conditionnements, désignés ci-après de A à D, ont en outre chacun des particularités résumées dans le tableau ci-dessous.

| Conditionnement | Particularités |
|---|---|
| A | - Présence d'une housse externe (film plastique) recouvrant la totalité des cartons |
| | - Absence de couloirs de refroidissement |
| B | - Absence de housse externe |
| | - Absence de couloirs de refroidissement |
| C | - Présence de housse externe |
| | - Présence de couloirs de refroidissement |
| D | - Absence de housse externe |
| | - Présence de couloirs de refroidissement |

### 2. Résultats

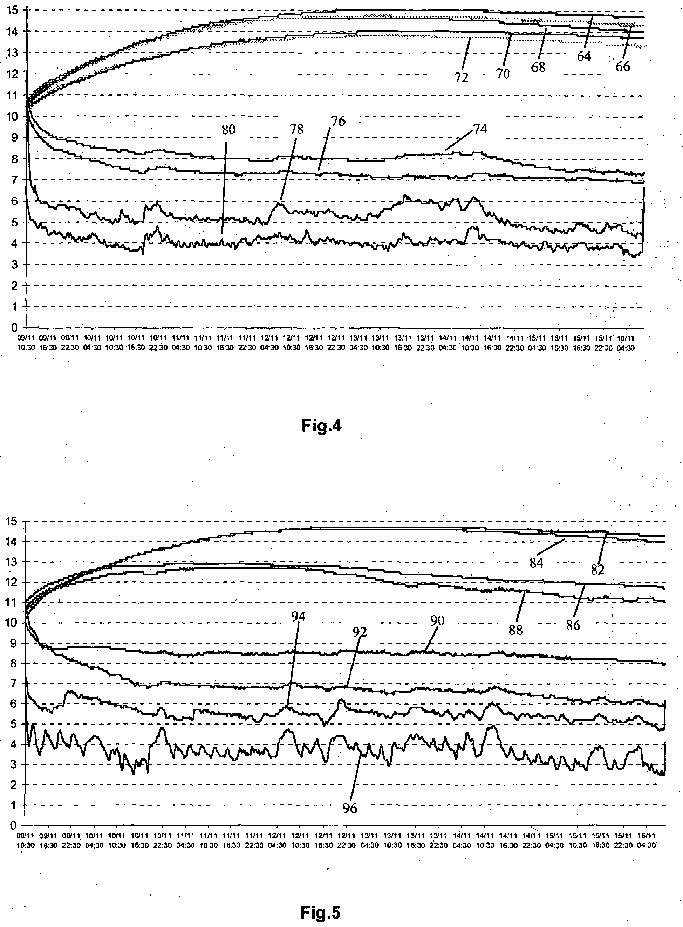

Les courbes d'évolution de la température en fonction du temps sont représentées sur les **figures 4 et 5****.** Le tableau suivant donne un récapitulatif des lieux où la température à été prise, afin de fournir les différentes courbes **des** **figures 4 et 5** **:**

| Référence sur les figures | Nature de la courbe |
|---|---|
| 64 | Température dans la 6^{ème} couche du conditionnement B |
| 66 | Température dans la 5^{ème} couche du conditionnement B |
| 70 | Température dans la 6^{ème} couche du conditionnement A |
| 72 | Température dans la 5^{ème} couche du conditionnement A |
| 74 | Température dans la 10^{ème} couche du conditionnement A |
| 76 | Température dans la 10^{ème} couche du conditionnement B |
| 78 | Température ambiante de la chambre froide du conditionnement A |
| 80 | Température ambiante de la chambre froide du conditionnement B |
| 82 | Température dans la 6^{ème} couche du conditionnement C |
| 84 | Température dans la 5^{ème} couche du conditionnement C |
| 86 | Température dans la 6^{ème} couche du conditionnement D |
| 88 | Température dans la 5^{ème} couche du conditionnement D |
| 90 | Température dans la 10^{ème} couche du conditionnement C |
| 92 | Température dans la 10^{ème} couche du conditionnement D |
| 94 | Température ambiante de la chambre froide du conditionnement C |
| 96 | Température ambiante de la chambre froide du conditionnement D |

On observe de manière générale un refroidissement moins efficace qu'avec les conditionnements de l'exemple 1, au niveau des couches intermédiaires.

La présence d'une housse externe sur un conditionnement sans couloir de refroidissement ne semble pas provoquer d'échauffement supplémentaire. Cependant, on observe une altération du carton due à un excès d'humidité à l'intérieur de la housse.

Pour les conditionnements avec couloirs de refroidissement, le film plastique empêche la ventilation au coeur du conditionnement et provoque un échauffement insatisfaisant.

## Revendications

1. Conditionnement (10) de produits solides de levure sous forme de levure pressée, comprenant :
- un élément de support (12) ;
- des produits solides de levure contenus dans un ou plusieurs emballages (22), supportés par l'élément de support (12) ;
- une housse externe (14) maintenant l'ensemble des produits solides de levure contenus dans les emballages (22) sur l'élément de support (12) ;
**caractérisé en ce que**:
- la totalité des emballages (22) présents entre les produits solides de levure et la housse externe (14) ont une masse surfacique inférieure ou égale à 150 g/m² et sont en partie perméables aux gaz et aux liquides, et
- les emballages (22) sont constitués de matériaux choisis parmi le cellophane, le papier, le film plastique alimentaire et le textile.

2. Conditionnement (10) selon la revendication 1, dans lequel la totalité des emballages (22) présents entre les produits solides de levure et la housse externe (14) ont :
- une masse surfacique inférieure ou égale à 100 g/m², et de manière plus particulièrement préférée inférieure ou égale à 80 g/m² ; et / ou
- une épaisseur inférieure ou égale à 150 µm, de préférence inférieure ou égale à 120 µm, de manière plus particulièrement préférée inférieure ou égale à 90 µm.

3. Conditionnement (10) selon l'une des revendications 1 ou 2, dans lequel la housse externe (14) est constituée d'un matériau plastique étirable, de préférence du polyéthylène mono-étiré ou bi-étiré.

4. Conditionnement (10) selon l'une des revendications 1 à 3, dans lequel les produits solides de levure sont regroupés en cartouches (26), chaque cartouche (26) étant contenue dans au moins un emballage (22), et les cartouches (26) sont de préférence regroupées en lots (28), les cartouches (26) de chaque lot (28) étant de préférence attachées entre elles, de préférence au moyen d'une ou plusieurs bandes de liaison (30).

5. Conditionnement (10) selon la revendication 4, dans lequel :
- des espaces (32) sont ménagés entre au moins une partie des lots (28) ; ou
- l'ensemble des lots (28) sont disposés sans espace entre eux.

6. Conditionnement (10) selon la revendication 4 ou 5, dans lequel les lots (28) sont disposés sur l'élément de support (12) en plusieurs couches superposées.

7. Conditionnement (10) selon la revendication 6 comprenant cinq couches, dans lequel :
- chaque couche comprend dix lots (28) ;
- chaque lot (28) comprend six cartouches (26), de préférence réparties en trois rangées de deux cartouches (26), les cartouches (26) de chaque lot (28) étant de préférence attachées par deux bandes de liaison (30) sensiblement parallèles et deux autres bandes de liaison (30) sensiblement parallèles qui croisent les précédentes ; et
- chaque cartouche (26) comprend cinq produits solides de levure.

8. Conditionnement (10) selon l'une des revendications 1 à 7, dans lequel les produits solides de levure sont de la levure pressée comprenant de 27 à 34% de matière sèche.

9. Procédé de conditionnement de produits solides de levure comprenant les étapes suivantes :
- la fourniture d'un élément de support (12) ;
- l'emballage de produits solides de levure, sous forme de levure pressée , dans un ou plusieurs emballages (22) ;
- la pose sur l'élément de support (12) des produits solides de levure contenus dans les emballages (22) ;
- la pose d'une housse externe (14) autour de l'ensemble des produits solides de levure contenus dans les emballages (22) ;
**caractérisé en ce que** :
- la totalité des emballages (22) présents entre les produits solides de levure et la housse externe (14) ont une masse surfacique inférieure ou égale à 150 g/m² et sont en partie perméables aux gaz et aux liquides, et
- les emballages (22) sont constitués de matériaux choisis parmi le cellophane, le papier, le film plastique alimentaire et le textile.

10. Procédé selon la revendication 9, dans lequel la totalité des emballages (22) présents entre les produits solides de levure et la housse externe (14) ont :
- une masse surfacique inférieure ou égale à 100 g/m², et de maniéré plus particulièrement préférée inférieure ou égale à 80 g/m²; et / ou
- une épaisseur inférieure ou égale à 150 µm, de préférence inférieure ou égale à 120 µm, de manière plus particulièrement préférée inférieure ou égale à 90 µm.

11. Procédé selon la revendication 9 ou 10, dans lequel :
- les emballages (22) sont constitués de matériaux choisis parmi le cellophane et le papier ; et / ou
- la housse externe (14) est constituée d'un matériau plastique étirable, de préférence du polyéthylène mono-étiré ou bi-étiré.

12. Procédé selon l'une des revendications 9 à 11, comprenant, avant l'étape de pose sur l'élément de support (12) des produits solides de levure contenus dans les emballages (22) :
- une étape de regroupement des produits solides de levure en cartouches (26), chaque cartouche étant contenue dans au moins un emballage (22) ; et
- une étape de regroupement des cartouches (26) en lots (28), les cartouches (26) de chaque lot (28) étant de préférence attachées entre elles, de préférence au moyen d'une ou plusieurs bandes de liaison (30) ;
et dans lequel, de préférence, l'étape de pose sur l'élément de support (12) des produits solides de levure contenus dans les emballages (22) comprend la pose des lots (28) sur l'élément de support (12) en couches superposées.

13. Procédé selon la revendication la revendication 12 dans lequel, lors de la pose sur l'élément de support (12) des produits solides de levure contenus dans les emballages (22) :
- des espaces (32) sont ménagés entre les lots (28) ; ou
- l'ensemble des lots (28) sont disposés sans espace entre eux.

14. Procédé de conditionnement selon l'une des revendications 9 à 13, dans lequel les produits solides de levure sont de la levure pressée comprenant de 27 à 34% de matière sèche.

15. Procédé de conservation et d'utilisation de produits solides de levure comprenant les étapes suivantes :
- conditionnement de produits solides de levure, sous forme de levure pressée, dans un conditionnement (10) selon l'une des revendications 1 à 8, de préférence selon un procédé de conditionnement selon l'une des revendications 10 à 14 ;
- conservation dudit conditionnement (10) de produits solides de levure jusqu'à son utilisation à une température extérieure inférieure ou égale à 15°C, préférentiellement inférieure ou égale à 10°C, et de manière plus particulièrement préférée inférieure ou égale à 5°C ;
- utilisation d'au moins un des produits solides de levure.

16. Procédé selon la revendication 15, dans lequel les produits solides de levure sont transportés au cours de l'étape de conservation ; et dans lequel l'étape de conservation présente de préférence une durée comprise entre 1 jour et 7 semaines, de préférence entre 1 semaine et 7 semaines.

17. Procédé selon la revendication 15 ou 16, dans lequel la température de chaque produit solide de levure reste inférieure ou égale à 15°C, de préférence inférieure ou égale à 12°C, et de manière plus particulièrement préférée inférieure ou égale à 10°C lors de l'étape de conservation.

18. Procédé selon l'une des revendications 15 à 17, dans lequel au moins un produit solide de levure est utilisé pour la fabrication d'un produit de panification.

## Patentansprüche

1. Verpackung (10) von festen Hefeprodukten in Form von Presshefe, umfassend:
- ein Trägerelement (12);
- feste Hefeprodukte, die in einer oder mehreren von dem Trägerelement (12) getragenen Packungen (22) enthalten sind;
- eine äußere Hülle (14), die die gesamten festen Hefeprodukte, welche in den Packungen (22) enthalten sind, auf dem Trägerelement (12) hält;
**dadurch gekennzeichnet, dass**:
- die gesamten Packungen (22), die zwischen den festen Hefeprodukten und der äußeren Hülle (14) vorhanden sind, eine flächenbezogene Masse kleiner oder gleich 150 g/m² aufweisen und teilweise gas- und flüssigkeitsdurchlässig sind, und
- die Packungen (22) aus Materialien bestehen, die aus Zellglas, Papier, Kunststofffolie und Textilien ausgewählt sind.

2. Verpackung (10) nach Anspruch 1, bei der die gesamten Packungen (22), die zwischen den festen Hefeprodukten und der äußeren Hülle (14) vorhanden sind:
- eine flächenbezogene Masse kleiner oder gleich 100 g/m² und stärker bevorzugt kleiner oder gleich 80 g/m²; und/oder
- eine Dicke kleiner oder gleich 150 µm, bevorzugt kleiner oder gleich 120 µm, stärker bevorzugt kleiner oder gleich 90 µm
aufweisen.

3. Verpackung (10) nach einem der Ansprüche 1 oder 2, bei der die äußere Hülle (14) aus einem verstreckbaren Kunststoffmaterial besteht, vorzugsweise aus monoaxial oder biaxial verstrecktem Polyethylen.

4. Verpackung (10) nach einem der Ansprüche 1 bis 3, bei der die festen Hefeprodukte in Kapseln (26) zusammengefasst sind, wobei jede Kapsel (26) in mindestens einer Packung (22) enthalten ist und die Kapseln (26) vorzugsweise in Einheiten (28) zusammengefasst sind, wobei die Kapseln (26) jeder Einheit (28) vorzugsweise mittels eines oder mehrerer Verbindungsbänder (30) aneinander befestigt sind.

5. Verpackung (10) nach Anspruch 4, bei der:
- Abstände (32) zwischen mindestens einem Teil der Einheiten (28) ausgebildet sind; oder
- die gesamten Einheiten (28) ohne Abstand dazwischen angeordnet sind.

6. Verpackung (10) nach Anspruch 4 oder 5, bei der die Einheiten (28) auf dem Trägerelement (12) in mehreren übereinanderliegenden Schichten angeordnet sind.

7. Verpackung (10) nach Anspruch 6, umfassend fünf Schichten, bei der:
- jede Schicht zehn Einheiten (28) umfasst;
- jede Einheit (28) sechs Kapseln (26) umfasst, die vorzugsweise in drei Reihen von zwei Kapseln (26) verteilt sind, wobei die Kapseln (26) jeder Einheit (28) vorzugsweise durch zwei im Wesentlichen parallele Verbindungsbänder (30) und zwei andere im Wesentlichen parallele Verbindungsbänder (30), die die vorgenannten kreuzen, befestigt sind; und
- jede Kapsel (26) fünf feste Hefeprodukte (26) umfasst.

8. Verpackung (10) nach einem der Ansprüche 1 bis 7, bei der die festen Hefeprodukte Presshefe mit 27 bis 34% Trockenmasse sind.

9. Verfahren zum Verpacken fester Hefeprodukte, umfassend die folgenden Schritte:
- Bereitstellen eines Trägerelements (12);
- Abpacken fester Hefeprodukte in Form von Presshefe in einer oder mehreren Packungen (22);
- Anordnen der in den Packungen (22) enthaltenen festen Hefeprodukte auf dem Trägerelement (12);
- Anordnen einer äußeren Hülle (14) um die gesamten in den Packungen (22) enthaltenen festen Hefeprodukte;
**dadurch gekennzeichnet, dass**:
- die gesamten zwischen den festen Hefeprodukten und der äußeren Hülle (14) vorhandenen Packungen (22) eine flächenbezogene Masse kleiner oder gleich 150 g/m² aufweisen und teilweise gas- und flüssigkeitsdurchlässig sind, und
- die Packungen (22) aus Materialien bestehen, die aus Zellglas, Papier, Kunststofffolie und Textilien ausgewählt sind.

10. Verfahren nach Anspruch 9, bei dem die gesamten zwischen den festen Hefeprodukten und der äußeren Hülle (14) vorhandenen Packungen (22):
- eine flächenbezogene Masse kleiner oder gleich 100 g/m² und stärker bevorzugt kleiner oder gleich 80 g/m²; und/oder
- eine Dicke kleiner oder gleich 150 µm, bevorzugt kleiner oder gleich 120 µm, stärker bevorzugt kleiner oder gleich 90 µm
aufweisen.

11. Verfahren nach Anspruch 9 oder 10, bei dem:
- die Packungen (22) aus Materialien bestehen, die aus Zellglas und Papier ausgewählt sind; und/oder
- die äußere Hülle (14) aus einem verstreckbaren Kunststoffmaterial besteht, vorzugsweise aus monoaxial oder biaxial verstrecktem Polyethylen.

12. Verfahren nach einem der Ansprüche 9 bis 11, das vor dem Schritt des Anordnens der in den Packungen (22) enthaltenen festen Hefeprodukte auf dem Trägerelement (12) umfasst:
- einen Schritt des Zusammenfassens der festen Hefeprodukte in Kapseln (26), wobei jede Kapsel in mindestens einer Packung (22) enthalten ist; und
- einen Schritt des Zusammenfassens der Kapseln (26) in Einheiten (28), wobei die Kapseln (26) jeder Einheit (28) vorzugsweise aneinander befestigt sind, bevorzugt mittels eines oder mehrerer Verbindungsbänder (30);
und bei dem der Schritt des Anordnens der in den Packungen (22) enthaltenen festen Hefeprodukte auf dem Trägerelement (12) vorzugsweise das Anordnen der Einheiten (28) auf dem Trägerelement (12) in übereinanderliegenden Schichten umfasst.

13. Verfahren nach Anspruch 12, bei dem beim Anordnen der in den Packungen (22) enthaltenen festen Hefeprodukte auf dem Trägerelement (12):
- Abstände (32) zwischen den Einheiten (28) ausgebildet werden; oder
- die gesamten Einheiten (28) ohne Abstand dazwischen angeordnet werden.

14. Verfahren zum Verpacken nach einem der Ansprüche 9 bis 13, bei dem die festen Hefeprodukte Presshefe mit 27 bis 34% Trockenmasse sind.

15. Verfahren zum Aufbewahren und Verwenden fester Hefeprodukte, umfassend die folgenden Schritte:
- Verpacken fester Hefeprodukte in Form von Presshefe in einer Verpackung (10) nach einem der Ansprüche 1 bis 8, vorzugsweise nach einem Verfahren zum Verpacken nach einem der Ansprüche 10 bis 14;
- Aufbewahren der Verpackung (10) von festen Hefeprodukten bis zu ihrer Verwendung bei einer Außentemperatur kleiner oder gleich 15°C, bevorzugt kleiner oder gleich 10°C und stärker bevorzugt kleiner oder gleich 5°C;
- Verwenden mindestens eines der festen Hefeprodukte.

16. Verfahren nach Anspruch 15, bei dem die festen Hefeprodukte im Verlauf des Aufbewahrungsschritts transportiert werden; und bei dem der Aufbewahrungsschritt vorzugsweise zwischen 1 Tag und 7 Wochen, bevorzugt zwischen 1 Woche und 7 Wochen, dauert.

17. Verfahren nach Anspruch 15 oder 16, bei dem die Temperatur jedes festen Hefeprodukts während des Aufbewahrungsschritts kleiner oder gleich 15°C, bevorzugt kleiner oder gleich 12°C und stärker bevorzugt kleiner oder gleich 10°C, bleibt.

18. Verfahren nach einem der Ansprüche 15 bis 17, bei dem mindestens ein festes Hefeprodukt zur Herstellung eines Brotproduktes verwendet wird.

## Claims

1. A package (10) of solid yeast products in compressed yeast form comprising:
- a support element (12);
- solid yeast products contained in one or more packagings (22), supported by the support element (12);
- an outer covering (14) maintaining the whole solid yeast products contained in the packagings (22) on the support element (12);
**characterized in that**
- the totality of the packagings (22) present between the solid yeast products and the outer covering (14) has a mass per unit surface area less than or equal to 150 g/m² and is partially gas-permeable and liquid-permeable, and
- the packagings (22) are made of materials chosen from cellophane, paper, food-grade plastic film, and textile.

2. The package (10) according to claim 1, wherein the totality of the packagings (22) present between the solid yeast products and the outer covering (14) has:
- a mass per unit surface area less than or equal to 100 g/m², and more particularly preferably less than or equal to 80 g/m²; and/or
- a thickness less than or equal to 150 µm, preferably less than or equal to 120 µm, more particularly preferably less than or equal to 90 µm.

3. The package (10) according to any one of claims 1 to 2, wherein the outer covering (14) is made of a stretchable plastic material, preferably single-stretched or double-stretched polyethylene.

4. The package (10) according to any one of claims 1 to 3, wherein the solid yeast products are grouped in packs (26), each pack (26) being contained in at least one packaging (22), and the packs (26) are preferably grouped into batches (28), the packs (26) of each batch (28) being preferably tied between them, preferably by means of one or more connecting bands (30).

5. The package (10) according to claim 4, wherein:
- spaces (32) are provided between at least some of the batches (28); or
- the whole batches (28) are arranged without spaces between them.

6. The package (10) according to claim 4 or 5, wherein the batches (28) are arranged on the support element (12) in a plurality of superimposed layers.

7. The package (10) according to claim 6, comprising five layers, wherein:
- each layer comprises ten batches (28);
- each batch (28) comprises six packs (26), preferably distributed in three rows of two packs (26), the packs (26) of each batch (28) being preferably tied by two connecting bands (30) essentially parallel and two other connecting bands (30) essentially parallel which cross the preceding ones; and
- each pack (26) comprises five solid yeast products.

8. The package (10) according to any one of claims 1 to 7, wherein the solid yeast products are compressed yeast comprising 27% to 34% of dry matter.

9. A method of packaging solid yeast products, comprising the following steps:
- the provision of a support element (12);
- the packaging of solid yeast products, in compressed yeast form, in one or more packagings (22);
- the placing on the support element (12) of the solid yeast products contained in the packagings (22);
- the placing of an outer covering (14) around the whole solid yeast products contained in the packagings (22) ;
**characterized in that**
- the totality of the packaging (22) present between the solid yeast products and the outer covering (14) has a mass per unit surface area less than or equal to 150 g/m² and is partially gas-permeable and liquid-permeable, and
- the packagings (22) are made of materials chosen from cellophane, paper, food-grade plastic film, and textile.

10. The method according to claim 9, wherein the totality of the packagings (22) present between the solid yeast products and the outer covering (14) have:
- a mass per unit surface area less than or equal to 100 g/m², and more particularly preferably less than or equal to 80 g/m²; and/or
- a thickness less than or equal to 150 µm, preferably less than or equal to 120 µm, and more particularly preferably less than or equal to 90 µm.

11. The method according to claim 9 or 10, wherein:
- the packagings (22) are made of materials chosen from cellophane and paper; and/or
- the outer covering (14) is made of a stretchable plastic material, preferably single-stretched or double-stretched polyethylene.

12. The method according to any one of claims 9 to 11, comprising, before the step of placing on the support element (12) the solid yeast products contained in the packagings (22):
- a step of grouping the solid yeast products into packs (26), each pack being contained in at least one packaging (22); and
- a step of grouping the packs (26) into batches (28), the packs (26) of each batch (28) being preferably tied between them, preferably by means of one or more connecting bands (30);
and wherein, preferably, the step of placing on the support element (12) the solid yeast products contained in the packagings (22) comprises the placing of the batches (28) on the support element (12) in superimposed layers.

13. The method according to claim 12, wherein, when placing on the support element (12) the solid yeast products contained in the packagings (22),
- spaces (32) are provided between the batches (28); or
- the whole batches (28) are arranged without spaces between them.

14. The packaging method according to any one of claims 9 to 13, wherein the solid yeast products are compressed yeast comprising 27% to 34% of dry matter.

15. A method of storing and using solid yeast products, comprising the following steps:
- packaging solid yeast products in a package (10) according to any one of claims 1 to 8, preferably by a packaging method according to any one of claims 10 to 14;
- storing said package (10) of solid yeast products, until their use, at an external temperature less than or equal to 15°C, preferably less than or equal to 10°C, and more particularly preferably less than or equal to 5°C;
- using at least one of the solid yeast products.

16. The method according to claim 15, wherein the solid yeast products are transported during the storage step, and wherein the storage step has preferably a duration comprised between 1 day and 7 weeks, preferably between 1 week and 7 weeks.

17. The method according to claim 15 or 16, wherein the temperature of each solid yeast product remains less than or equal to 15°C, preferably less than or equal to 12°C, and more particularly preferably less than or equal to 10°C during the storage step.

18. The method according to any one of claims 15 to 17, wherein at least one solid yeast product is used for the production of a bread-making product.
